# EUROPEAN PATENT SPECIFICATION

(11) **EP 4 306 115 B1**
(45) Date of publication and mention of the grant of the patent: **05.08.2026**
(21) Application number: 22836841.1
(22) Date of filing: 04.07.2022
(51) Int. Cl.: A61K 31/675, A61K 9/20, A61K 9/26, A61P 35/00

(54) **PHARMACEUTICAL COMPOSITION, AND PREPARATION METHOD THEREFOR AND APPLICATION THEREOF**
PHARMAZEUTISCHE ZUSAMMENSETZUNG UND HERSTELLUNGSVERFAHREN DAFÜR UND ANWENDUNG DAVON
COMPOSITION PHARMACEUTIQUE, SON PROCÉDÉ DE PRÉPARATION ET SON APPLICATION

(30) Priority: 05.07.2021 CN 202110757316; 13.06.2022 CN 202210660192
(43) Date of publication of application: 17.01.2024
(73) Proprietor: QILU PHARMACEUTICAL CO., LTD., Jinan, Shandong 250100 (CN)
(72) Inventor: ZHENG, Xiaoqing, Jinan, Shandong 250100 (CN); CHENG, Lizhen, Jinan, Shandong 250100 (CN); GENG, Weifeng, Jinan, Shandong 250100 (CN); YANG, Qingmin, Jinan, Shandong 250100 (CN)
(74) Representative: Graf von Stosch Patentanwaltsgesellschaft mbH
(86) International application number: PCT/CN2022/103576
(87) International publication number: WO 2023/280090

(56) References cited:
- EP-A1- 3 165 530
- WO-A1-2016/000581
- CN-A- 105 330 698
- CN-A- 106 176 752
- CN-A- 106 187 915
- CN-A- 106 928 275
- CN-A- 106 928 275
- CN-A- 108 721 243
- CN-A- 110 407 877
- CN-A- 110 407 877
- KYUNG ROK CHU ET AL: "Effect of particle size on the dissolution behaviors of poorly water-soluble drugs", ARCHIVES OF PHARMACAL RESEARCH, PHARMACEUTICAL SOCIETY OF KOREA, HEIDELBERG, vol. 35, no. 7, 3 August 2012 (2012-08-03), pages 1187 - 1195, XP035094775, ISSN: 1976-3786, DOI: 10.1007/S12272-012-0709-3
- LIU CHAO, ZONG JIANFEI;QINGZHOU YAO WANG: "Analysis on Factors Affecting Tablet Dissolution", CHINESE JOURNAL OF PHARMACEUTICALS, vol. 50, no. 3, 1 April 2019 (2019-04-01), CN , pages 252 - 259, XP093020586, ISSN: 1001-8255, DOI: 10.16522/j.cnki.cjph.2019.03.002
- "Dissolution of the Pharmaceutical Solid Formulation", 31 October 1994, PEOPLE'S HEALTH PUBLISHING HOUSE, CN, ISBN: 9787117020534, article WU, GUANGCHEN : " Factors Affecting Tablet Dissolution", pages: 69 - 72, XP009542338

## Description

### TECHNICAL FIELD

The present invention belongs to the technical field of pharmaceutical chemistry, and relates to a pharmaceutical composition and preparation method and use thereof.

### BACKGROUND

Spirocyclic aryl phosphorus oxide, with a structure represented by the following formula, is chemically named as: (2-((5-chloro-2-((2-methoxy-4-(9-methyl-3,9-diazaspiro[5.5]undecan-3-yl)phenyl)amino)pyrimidin-4-yl)amino)phenyl)dimethylphosphine oxide, which has a molecular weight of 569.08, and is a highly selective anaplastic lymphoma kinase (ALK) inhibitor. Its inhibitory effect on the enzyme activity of major kinase targets including ALK wild type, ALK mutant (ALK^{L1196M}, ALK^{C1156Y}) and EGFR mutant EGFR^{T790M/L858R} were determined at molecular level. Spirocyclic aryl phosphorus oxide showed similar or slightly better inhibitory activity to 4 major kinase targets compared with AP26113. Low inhibitory activity to other related serine, threonine and tyrosine proteases except EGFR wild type was observed in the detected concentration range, showing high selectivity to kinase. According to mechanism of action and results of preclinical trials, spirocyclic aryl phosphorus oxide can be used in the treatment of ALK positive non-small cell lung cancer patients with progressive disease after treatment with Crizotinib or unable to tolerate Crizotinib, or ALK positive non-small cell lung cancer patients who have not used ALK inhibitors.

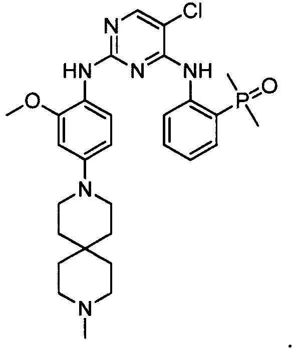

Spirocyclic aryl phosphorus oxide is white or off-white powder with almost no hygroscopicity, and is easily soluble in dichloromethane, sparingly soluble in methanol, slightly soluble in water, ethanol or acetonitrile, and practically insoluble, or insoluble in 1mol/L sodium hydroxide solution. However, spirocyclic aryl phosphorus oxide is easily soluble in 1mol/L hydrochloric acid solution and is a compound of high solubility. The solubility of the drug directly influences the dissolution rate of the composition containing the drug, which in turn influences the bioavailability of the pharmaceutical composition. Therefore, how to improve the dissolution rate of pharmaceutical compositions containing spirocyclic aryl phosphorus oxide in solvents other than hydrochloric acid has become an urgent problem to be solved.

EP3165530A1 discloses a spirocyclic aryl phosphorus oxide or sulfide as an ALK inhibitor.

CN110407877A discloses polymorphs of (2-((5-chloro-2-((2-methoxy-4-(9-methyl-3,9-diazaspiro[5.5]undecan-3-yl)phenyl)amino)pyrimidin-4-yl)amino)phenyl)dimethylphosphine oxide, and a pharmaceutical composition comprising the same.

CN106928275A discloses a crystal form of high-purity spirocyclic aryl phosphorus oxide.

Kyung Rok Chu et al. ("Effect of particle size on the dissolution behaviors of poorly water-soluble drugs", Archives of Pharmacal Research, Vol. 35, No. 7, August 03, 2012, pages 1187-1195) discloses a study examined the effects of the particle size of various poorly water-soluble drugs on their dissolution behavior through physicochemical and mathematical analysis.

### SUMMARY

The invention is set out in the appended set of claims.

It is known in the art that the particle size of drugs generally has no significant effect on drug dissolution behavior. However, the inventors of the present application found that although the particle size of spirocyclic aryl phosphorus oxide has a significant effect on the drug dissolution in vitro, too large or too small particle size will lead to a decrease in dissolution of spirocyclic aryl phosphorus oxides. Only by controlling the particle size of spirocyclic aryl phosphorus oxide within a certain range, the drug dissolution can be ensured to meet the requirements. Therefore, in order to improve the dissolution rate of the spirocyclic aryl phosphorus oxide or a pharmaceutically acceptable salt thereof, the range of particle size should be properly controlled so as to effectively control the dissolution rate of the spirocyclic aryl phosphorus oxide in formulations containing spirocyclic aryl phosphorus oxide, and further improve the bioavailability of the spirocyclic aryl phosphorus oxide in vivo.

In view of this, the object of the present invention is to provide a pharmaceutical composition and preparation method and use thereof. The spirocyclic aryl phosphorus oxide or a pharmaceutically acceptable salt thereof with a particular range of particle size can improve the dissolution rate of the pharmaceutical composition, and thus improve the bioavailability.

In a first aspect of the present invention, a spirocyclic aryl phosphorus oxide or a pharmaceutically acceptable salt thereof with a particle size distribution satisfying D90 in a range of 40.3 µm to 79.6 µm is provided, and the spirocyclic aryl phosphorus oxide has the following structure:

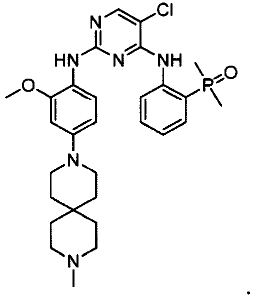

In a second aspect of the present invention, a spirocyclic aryl phosphorus oxide or a pharmaceutically acceptable salt thereof with the particle size distribution satisfying D50 in the range of 13.0 µm to 23.8 µm is provided, or a spirocyclic aryl phosphorus oxide or a pharmaceutically acceptable salt thereof with the particle size distribution satisfying D50 in the range of 13.0 µm to 18.2 µm is provided.

A third aspect of the present invention provides a pharmaceutical compositon comprising a spirocyclic aryl phosphorus oxide or a pharmaceutically acceptable salt thereof with particular specific particle size, and a pharmaceutically acceptable carrier, wherein the spirocyclic aryl phosphorus oxide or a pharmaceutically acceptable salt thereof is used as an active ingredient, the particular particle size satisfies D90 in the range of 40.3 µm to 79.6 µm, and the spirocyclic aryl phosphorus oxide has the following structure:

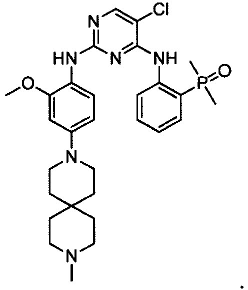

In some embodiments of the present invention, the D50 of the spirocyclic aryl phosphorus oxide or a pharmaceutically acceptable salt thereof is in the range of 13.0 µm to 23.8 µm.

In some embodiments of the present invention, the D50 of the spirocyclic aryl phosphorus oxide or a pharmaceutically acceptable salt thereof is in the range of 13.0 µm to 18.2 µm. In some embodiments of the present invention, the spirocyclic aryl phosphorus oxide in the pharmaceutical composition of the present invention is in crystal form A.

In some preferred embodiments, the crystal form A of the spirocyclic aryl phosphorus oxide in the pharmaceutical composition of the present invention has characteristic peaks in the X-ray powder diffraction spectrum at 2θ degrees of 8.5±0.20°, 10.1±0.20°, 12.1±0.20°, 14.6±0.20°, 17.7±0.20°, 18.7±0.20°, 20.4±0.20° and 21.2±0.20°, using Cu-Kα radiation.

In some preferred embodiments, the crystal form A of the spirocyclic aryl phosphorus oxide in the pharmaceutical composition of the present invention has characteristic peaks in the X-ray powder diffraction spectrum at 2θ degrees of 8.5±0.20°, 9.3±0.20°, 10.1±0.20°, 12.1±0.20°, 13.7±0.20°, 14.6±0.20°, 15.7±0.20°, 16.5±0.20°, 17.7±0.20°, 18.7±0.20°, 20.0±0.20°, 20.4±0.20°, 21.2±0.20°, 23.6±0.20°, 25.1±0.20° and 25.6±0.20°, using Cu-Kα radiation.

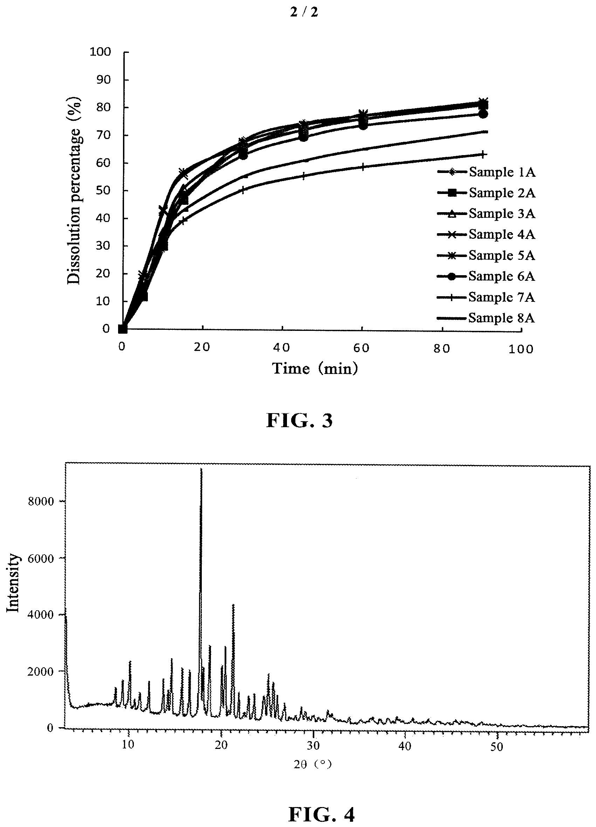

In some more preferred embodiments, the crystal form A of the spirocyclic aryl phosphorus oxide in the pharmaceutical composition of the present invention has an X-ray powder diffraction spectrum substantially as shown in FIG.4.

In some embodiments, the pharmaceutically acceptable carrier comprises a filler, a disintegrant, an adhesive, a glidant and a lubricant.

In some preferred embodiments, the filler is selected from the group consisting of starch, mannitol, sorbitol, microcrystalline cellulose, lactose, pregelatinized starch and inorganic salts or a combination thereof, preferably a combination of microcrystalline cellulose and pregelatinized starch. The inorganic salts may include calcium sulfate (containing two molecules of crystal water), calcium hydrogen phosphate, calcium carbonate, etc.

In some preferred embodiments, the disintegrant is selected from the group consisting of dry starch, sodium carboxymethyl starch, hydroxypropyl starch, low substituted hydroxypropyl cellulose, crospovidone and croscarmellose sodium or a combination thereof, preferably sodium carboxymethyl starch.

In some preferred embodiments, the adhesive is selected from the group consisting of distilled water, ethanol, starch paste, powdered sugar and syrup, hydroxypropyl methylcellulose, povidone, hydroxypropyl cellulose, methyl cellulose, ethyl cellulose and sodium carboxymethyl cellulose or a combination thereof, preferably hydroxypropyl methylcellulose.

In some preferred embodiments, the glidant is selected from the group consisting of micronized silica gel and talc or a combination thereof, preferably micronized silica gel.

In some preferred embodiments, the lubricant is selected from the group consisting of magnesium stearate, hydrogenated vegetable oil, polyethylene glycol, dodecyl magnesium sulfate and sodium stearyl fumarate or a combination thereof, preferably magnesium stearate.

In some embodiments, the pharmaceutical composition comprising the spirocyclic aryl phosphorus oxide or a pharmaceutically acceptable salt thereof of a particular particle size of the present invention is an oral preparation, which is absorbed via gastrointestinal tract through oral administration.

In some preferred embodiments, the pharmaceutical composition of the present invention is a tablet.

In some preferred embodiments, the pharmaceutical composition of the present invention is a film-coated tablet.

In some embodiments, for the pharmaceutical composition of the present invention, the spirocyclic aryl phosphorus oxide or a pharmaceutical acceptable salt thereof accounts for 1.0% to 50.0% by weight in the composition.

In some preferred embodiments, in the pharmaceutical composition of the present invention, the following components each in percentage by weight are: 1% to 50% of spirocyclic aryl phosphorus oxide or a pharmaceutically acceptable salt thereof, 1% to 90% of the filler, 1% to 10% of the disintegrant, 0.1% to 1% of the glidant and 0.5% to 5% of the lubricant.

In some more preferred embodiments, in the pharmaceutical composition of the present invention, the following components each in percentage by weight are: 5.0% of spirocyclic aryl phosphorus oxide or a pharmaceutically acceptable salt thereof, 26.0% of microcrystalline cellulose, 59.5% of pregelatinized starch, 5% of sodium carboxymethyl starch, 3.0% of hydroxypropyl methylcellulose, 0.5% of micronized silica gel and 1% of magnesium stearate.

A fourth aspect of the present invention provides a preparation method of the pharmaceutical composition of any one in the above embodiments, which comprises the following steps:
1) fully mixing a spirocyclic aryl phosphorus oxide or a pharmaceutically acceptable salt thereof with a portion of internal excipients including a fillers, an adhesive and a disintegrant, and then granulating;
2) adding other excipients including a filler, a disintegrant and a lubricant, and then fully mixing; and
3) tabletting and coating.

In some embodiments of the present invention, a pharmaceutical composition is provided, comprising an effective amount of a spirocyclic aryl phosphorus oxide or a pharmaceutically acceptable salt thereof and a pharmaceutically acceptable carrier, a diluent and an excipient. Pharmaceutical compositions can be formulated for particular administration routes, such as oral administration, parenteral administration, and rectal administration, etc. Oral administration may include oral, sublingual and buccal administration, and oral administration may be administered by tablets (film-coated tablets), capsules (including sustained or timed release prescription), pills, powders, granules, elixir, tinctures, suspensions (including nano-suspensions, micron suspensions, spray-dried dispersants), syrup or emulsions; parenteral administration may include injection, infusion, and intranasal or other topical administrations. Specifically, it may be administered by subcutaneous, intravenous, intramuscular or intrasternal injection; infusion techniques, such as aseptic injectable aqueous or non-aqueous solutions or suspensions; intranasal administration, including nasal mucosal administration, such as by inhalation spray; and topical administration, such as in the form of creams or ointments. Rectal administration is for example in the form of suppositories. The above administration routs can be administered separately, but usually with pharmaceutical carriers selected according to the selected administration route and standard pharmaceutical procedures.

A fifth aspect of the present invention provides a pharmaceutical composition of any one in the above embodiments or a pharmaceutical composition prepared by the method of any one in the above embodiments for use in the treatment of cancers. The cancers include non-small cell lung cancer, lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial carcinoma, lung cancer, hepatocellular carcinoma, gastric carcinoma, gastrointestinal stromal tumor (GIST), acute myeloid leukemia (AML), cholangiocarcinoma, renal carcinoma, thyroid cancer, anaplastic large cell lymphoma, mesothelioma, multiple myeloma and melanoma.

In some embodiments, the cancer is non-small cell lung cancer.

In some embodiments, the above use is for ALK positive non-small cell lung cancer patients with progressive disease after treatment with Crizotinib or unable to tolerate Crizotinib.

In some embodiments, the above use is for ALK positive non-small cell lung cancer patients who have not used ALK inhibitors.

A sixth aspect of the present invention provides a method of treatment of cancer, which comprises administering a therapeutically effective amount of the pharmaceutical composition of any one in the aforementioned embodiments to the patient. The cancers include non-small cell lung cancer, lymphoma, non-Hodgkin lymphoma, ovarian cancer, cervical cancer, prostate cancer, colorectal cancer, breast cancer, pancreatic cancer, glioma, glioblastoma, melanoma, leukemia, gastric cancer, endometrial carcinoma, lung cancer, hepatocellular carcinoma, gastric carcinoma, gastrointestinal stromal tumor, acute myeloid leukemia, cholangiocarcinoma, renal carcinoma, thyroid cancer, anaplastic large cell lymphoma, mesothelioma, multiple myeloma and melanoma.

In some embodiments, the above cancer is non-small cell lung cancer.

In some embodiments, the above method is used for ALK positive non-small cell lung cancer patients with progressive disease after treatment with Crizotinib or unable to tolerate Crizotinib.

In some embodiments, the above method is used for ALK positive non-small cell lung cancer patients who have not used ALK inhibitors.

In the present invention, D50 refers to the particle size corresponding to the cumulative volume distribution percentage of spirocyclic aryl phosphorus oxides or pharmaceutically acceptable salts thereof reaching 50% measured from a small particle size; D90 refers to the particle size corresponding to the cumulative volume distribution percentage of spirocyclic aryl phosphorus oxides or pharmaceutically acceptable salts thereof reaching 90% measured from a small particle size.

### Beneficial technical effects:

The present invention provides a pharmaceutical composition containing a spirocyclic aryl phosphorus oxide or a pharmaceutically acceptable salt thereof having a particular particle size range, which can increase the dissolution rate of the pharmaceutical composition and improve the bioavailability.

Unless otherwise stated, the following terms and phrases used herein are intended to have the following meanings. A particular term or phrase should not be considered indefinite or unclear in the absence of a particular definition, but should be understood according to its ordinary meaning.

The term "pharmaceutically acceptable" as used herein refers to those compounds, materials, compositions, and/or dosage forms which are, within the scope of sound medical judgment, suitable for contact with tissues of human beings or animals without excessive toxicity, irritation, allergic response, or other problems, or complications, and is commensurate with a reasonable benefit/risk ratio.

The term "pharmaceutically acceptable salt" refers to the derivatives prepared from the compounds of the present invention and relatively non-toxic acids or bases. These salts can be prepared during synthesis, separation and purification of compounds, or can be prepared solely by using the purified compound in the free form to react with suitable acids or bases. When the compound contains relatively acidic functional groups, it reacts with alkali metals, alkaline earth metal hydroxides or organic amines to obtain alkali addition salts, including cations based on alkali and alkaline earth metals, such as sodium, lithium, potassium, calcium, magnesium, etc., and non-toxic ammonium, quaternary ammonium and amine cations, including ammonium, tetramethylammonium, tetraethylammonium, methylamine, dimethylamine, trimethylamine, triethylamine, ethylamine, etc. Pharmaceutically acceptable salt also covers salts of amino acids, such as argininate, gluconate, galacturonate, etc. When a compound contains relatively basic functional groups, it reacts with organic or inorganic acids to obtain acid addition salts, including inorganic salts such as hydrochloride, hydrobromide, nitrate, carbonate, bicarbonate, phosphate, monophosphate, dihydrogen phosphate, sulfate, hydrogen sulfate, hydroiodate, hydrobromide, metaphosphate and pyrophosphate, or organic salts including, for example, acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, benzene sulfonate, toluenesulfonate, phenylacetate, citrate, lactate, maleate, tartrate, methanesulfonate, etc.

The term "pharmaceutically acceptable carrier" refers to a medium generally acceptable in the art for the delivery of bioactive agents to animals, particularly mammals, according the administration and property of formulation, including, for example, adjuvants, excipients or vehicles, such as fillers, disintegrants, adhesives, glidants, lubricants, diluents, preservatives, flow modulators, wetting agents, emulsifiers, suspending agents, sweeteners, flavors, fragrances, antibacterial agents, antifungal agents, lubricants and dispersants. Pharmaceutically acceptable carriers are formulated according to a quantity of factors within the knowledge of those skilled in the art. Such factors include the types and properties of the active agents to be formulated, the subjects to be administered with the composition containing such active agents, the expected administration routes of the composition, and the indications of target treatment. Pharmaceutically acceptable carriers include both aqueous and non-aqueous medium, as well as a variety of solid and semi-solid formulations. In addition to active agents, such carriers include many different ingredients and additives, and such additional ingredients included in prescriptions for a variety of reasons (such as stabilizing active agents, adhesives, etc.) are well known to those skilled in the art. The term "excipient" generally refers to the carrier, diluent, and/or medium required for the formulation of an effective pharmaceutical composition.

The term "therapeutically effective amount" refers to a sufficient amount of the pharmaceutical composition of the present invention to treat disorders in a reasonable effect/risk ratio suitable for any medical treatment. However, it should be recognized that the total daily dosage of the composition of the present invention must be determined by the attending physician within the scope of reliable medical judgment. For any specific patient, the specific therapeutically effective dose level shall depend on a variety of factors, including the disorder to be treated and the severity of the disorder; the activity of the specific compound used; the specific composition used; age, weight, general health condition, gender and diet of the patient; the administration time, route and excretion rate of the specific compound used; treatment duration; drugs used in combination with or simultaneously with the specific compounds used; and similar factors known in the medical field.

### BRIEF DESCRIPTION OF THE DRAWINGS

In order to explain the examples of the present invention and the technical solutions of the prior art more clearly, the following briefly introduces the drawings used in the examples and the prior art. Obviously, the drawings in the following description are only some examples of the present invention, and for those skilled in the art, other drawings may be obtained based on these drawings without any creative efforts.
FIG. 1 shows the X-ray powder diffraction spectrum of the remaining solid spirocyclic aryl phosphorus oxide of samples 1-8 in Table 2 in suspensions during solubility test;
FIG. 2 shows the comparison of the dissolution curves of examples 1-4 and comparative examples 1-4 in phosphate buffer at pH 6.8;
FIG. 3 shows the comparison of the dissolution curves of examples 1-4, and comparative examples 1-4 in purified water with 0.5% Tween 20 added;
FIG. 4 shows the X-ray powder diffraction spectrum of crystal form A of spirocyclic aryl phosphorus oxide.

### DETAILED DESCRIPTION

In order to make clear the purpose, technical solutions, and advantages of the present invention, the present invention is further described in detail with reference to the drawings and examples.

In the example of the present invention, the crystal form of spirocyclic aryl phosphorus oxide is crystal form A, which is a known and commercially available raw material, or can be synthesized by or in accordance with a method known in the art.

### Determination of solubility of spirocyclic aryl phosphorus oxide

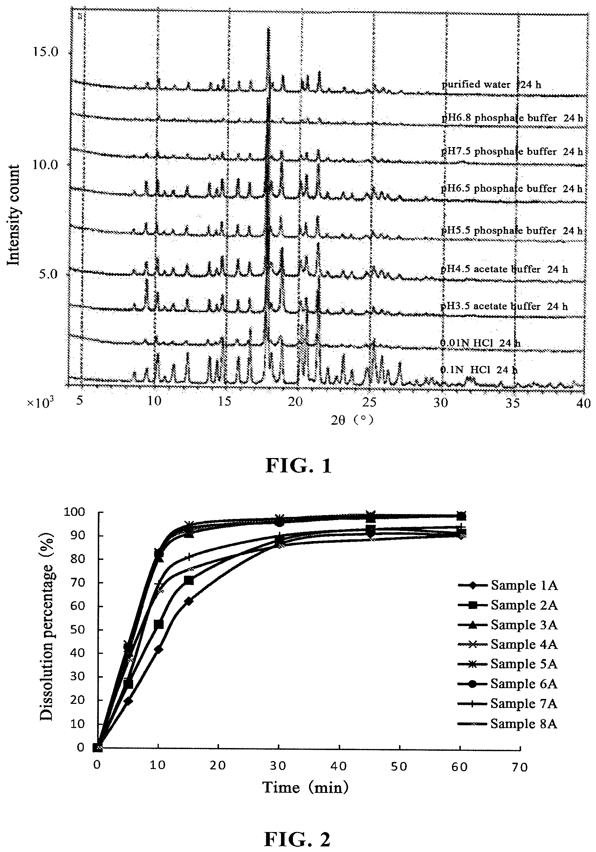

The solubilities of spirocyclic aryl phosphorus oxides in buffers at different pHs were determined. Nine test samples of 4 mg were each weighed and added to sample bottles of 1.5mL, and then different solvents (0.1 mol/L HCl, 0.01 mol/L HCl, pH3.5 acetate buffer, pH4.5 acetate buffer, pH5.5 phosphate buffer, pH6.5 phosphate buffer, pH6.8 phosphate buffer, pH7.5 phosphate buffer and water) of 1 mL were added. According to the dissolution, the raw material compounds were continuously added to form a saturated solution. A magneton was added to the saturated solution and then stirred on a magnetic agitator (temperature was 37 °C, away from light). After stirring for 24 hours, the samples were taken and centrifuged, and the residual solid samples in the lower layer were tested by X-ray powder diffraction (XRPD). The upper sample was filtered by a filter membrane, the final pH value of the filtrate was measured, and the saturated solubility of the compound was determined by high performance liquid chromatography (HPLC). The results of the determination are shown in Table 1, and the results of the XRPD are shown in FIG. 1.

**Table 1 Determination results of solubility of spirocyclic aryl phosphorus oxides in buffers with different pH values**

| **Solvents** | **Status (24 hrs)** | **Solubility (mg/mL)** | **Dose/solubility (mL, in 60mg specifications)** | **Conclusions** | **XRPD** |
|---|---|---|---|---|---|
| 0.1 mol/L HCl (pH 1.02) | yellow suspension | 53.4 | 1.12 | soluble | no change |
| 0.01 mol/L HCl (pH2.02) | white suspension | 6.9 | 8.69 | slightly soluble | no change |
| pH 3.5 buffer (pH 3.53) | dark yellow solution | 279.4 | 0.22 | easily soluble | no change |
| pH 4.5 buffer (pH 4.49) | yellow suspension | 108.0 | 0.56 | easily soluble | no change |
| pH 5.5 buffer (pH 5.47) | white suspension | 7.1 | 8.45 | slightly soluble | no change |
| pH 6.5 buffer (pH 6.45) | white suspension | 11.1 | 5.41 | sparingly soluble | no change |
| pH 7.5 buffer (pH 7.53) | white suspension | 0.85 | 70.59 | very slightly soluble | no change |
| pH 6.8 buffer (pH 6.83) | white suspension | 3.6 | 16.67 | slightly soluble | no change |
| water (pH 6.33) | white suspension | 0.06 | >250 | practically insoluble | no change |

Note: the easily soluble, soluble, sparingly soluble, slightly soluble and very slightly soluble in Table 1 mean that 1g of spirocyclic aryl phosphorus oxides can be dissolved in 1 ml to less than 10 ml, 10 ml to less than 30 ml, 30 ml to less than 100 ml, 100 ml to less than 1000 ml, 1000 ml to less than 10000 ml of solvent, respectively, and practically insoluble means that 1g of spirocyclic aryl phosphorus oxides can not be completely dissolved in 10000 ml of solvent.

It can be seen from Table 1 that the solubility change of spirocyclic aryl phosphorus oxide in buffers at different pHs is related to pH, and the spirocyclic aryl phosphorus oxide can be soluble in 0.1mol/L hydrochloric acid solution, easily soluble in buffer at pH3.5 and buffer at pH4.5, slightly soluble in 0.01mol/L hydrochloric acid solution, buffer at pH5.5 and buffer at pH6.8, sparingly soluble in buffer at pH6.5, very slightly soluble in buffer at pH7.5, and practically insoluble in water. It can be seen from FIG. 1, the crystal form of spirocyclic aryl phosphorus oxide does not change in different solvents.

### Preparation and process of pharmaceutical compositions containing spirocyclic aryl phosphorus oxide with different particle sizes

The content uniformity and in vitro dissolution rate of pharmaceutical compositions containing the spirocyclic aryl phosphorus oxide with different particle sizes were tested.

**Table 2: Spirocyclic aryl phosphorus oxide samples with different particle sizes**

| Particle size | Sample 1 | Sample 2 | Sample 3 | Sample 4 | Sample 5 | Sample 6 | Sample 7 | Sample 8 |
|---|---|---|---|---|---|---|---|---|
| D90 (µm) | 11.6 | 27.1 | 40.3 | 62 | 69 | 79.6 | 122 | 193 |
| D50 (µm) | 5.4 | 10.1 | 13.0 | 23.8 | 16.1 | 18.2 | 47.9 | 63.1 |
| D10 (µm) | 0.7 | 1.3 | 1.6 | 5.0 | 1.8 | 2.3 | 6.8 | 7.8 |

**Table 3 Solid composition of spirocyclic aryl phosphorus oxide**

| **Prescription** | |
|---|---|
| Unit | mg/tablet |
| spirocyclic aryl phosphorus oxide | 60.0 |
| microcrystalline cellulose PH101 | 46.8 |
| pregelatinized starch | 110.4 |
| sodium carboxymethyl starch | 12.0 |
| hydroxypropyl methylcellulose | 7.2 |
| silicon dioxide | 1.2 |
| magnesium stearate | 2.4 |
| Total | 240 |

### Examples 1-4

### Preparation process:

Spirocyclic aryl phosphorus oxide samples 3-6 with different particle sizes in Table 2 were prepared according to the prescription in Table 3 and the following preparation steps to obtain different pharmaceutical compositions sample 3A, sample 4A, sample 5A and sample 6A:
adding spirocyclic aryl phosphorus oxides with different particle sizes, microcrystalline cellulose, pregelatinized starch, hydroxypropyl methylcellulose and half the prescription amount of sodium carboxymethyl starch to High shear mixer to mix for 5 min; and adding purified water to granulate with stirring for 8 min;
performing wet granulation by granulator, drying in fluidized bed and sieving by granulator;
adding the remaining sodium carboxymethyl starch and silicon dioxide to the particles and mixing; adding magnesium stearate, sieving, mixing, and tabletting.

### Comparative examples 1-4

Except that spirocyclic aryl phosphorus oxides were successively replaced with samples 1, 2, 7 and 8 shown in Table 2, the rest were the same as in example 1.

### Determination of content uniformity and dissolution rate of pharmaceutical compositions in various examples and comparative examples

### 1) Detection of content uniformity

The content uniformity test of the pharmaceutical composition of each example and comparative example refers to the "Appendix XE content uniformity test method" of Chinese Pharmacopoeia 2015 edition, and the results are shown in Table 4.

**Table 4 Content Uniformity**

| | | **Average content (%)** | Content Uniformity (A+2.20S≤15.0) |
|---|---|---|---|
| Example 1 | Sample 3A | 100.0 | 4.7 |
| Example 2 | Sample 4A | 100.1 | 6.1 |
| Example 3 | Sample 5A | 98.8 | 5.9 |
| Example 4 | Sample 6A | 99.7 | 4.5 |
| Comparative example 1 | Sample 1A | 98.9 | 5.2 |
| Comparative example 2 | Sample 2A | 99.8 | 4.6 |
| Comparative example 3 | Sample 7A | 99.4 | 5.4 |
| Comparative example 4 | Sample 8A | 99.0 | 4.3 |

According to the results of content uniformity, the content uniformity of the pharmaceutical compositions can meet the requirements of quality standards when the particle size of raw materials is not more than 200 µm and not less than 10 µm.

### 2) Dissolution detection

The dissolution performance of the pharmaceutical composition in each example and comparative example was tested according to the dissolution determination method (Chinese Pharmacopoeia 2015 edition IV 0512), and specifically, the rotational speed was 50 rpm, maintained at 37±0.5 °C. The dissolution curve of the pharmaceutical composition was investigated, and the dissolution data are shown in Tables 5 and 6 below.

**Table 5 Dissolution data of spirocyclic aryl phosphorus oxide solid composition in phosphate buffer at pH6.8**

| | **Sample number** | **Comparison of dissolution curves (%)** | | | | | |
|---|---|---|---|---|---|---|---|
| | | 5min | 10min | 15min | 30min | 45min | 60min |
| Example 1 | Sample 3A | 40.5 | 80.7 | 91.2 | 96.8 | 98.2 | 99.4 |
| Example 2 | Sample 4A | 42.3 | 82.9 | 92.6 | 97.6 | 99.7 | 99.7 |
| Example 3 | Sample 5A | 43.6 | 83.1 | 94.7 | 97.8 | 99.2 | 99.6 |
| Example 4 | Sample 6A | 42.7 | 82.6 | 93.4 | 96.1 | 98.7 | 99.3 |
| Comparative example 1 | Sample 1A | 19.7 | 41.7 | 62.4 | 86.9 | 91.5 | 91.2 |
| Comparative example 2 | Sample 2A | 26.9 | 52.5 | 71.2 | 88.6 | 93.4 | 92.1 |
| Comparative example 3 | Sample 7A | 29.4 | 69.7 | 81.2 | 90.4 | 93.4 | 94.7 |
| Comparative example 4 | Sample 8A | 37 | 66.5 | 75.9 | 86.1 | 89 | 91 |

**Table 6 Dissolution data of spirocyclic aryl phosphorus oxide solid composition in purified water + 0.5% Tween 20**

| | **Sample number** | **Comparison of dissolution curves (%)** | | | | | | |
|---|---|---|---|---|---|---|---|---|
| | | 5min | 10min | 15min | 30min | 45min | 60min | 90min |
| Example 1 | Sample 3A | 14.2 | 35.2 | 51 | 65.4 | 74.2 | 76.5 | 82.7 |
| Example 2 | Sample 4A | 18.5 | 43.3 | 55.7 | 67.8 | 74.1 | 77.8 | 83 |
| Example 3 | Sample 5A | 19.7 | 42.6 | 56.8 | 66.3 | 72.5 | 78.4 | 84 |
| Example 4 | Sample 6A | 15.2 | 32.4 | 48.6 | 63.1 | 69.8 | 74.2 | 78.7 |
| Comparative example 1 | Sample 1A | 12 | 29.2 | 46.7 | 68.3 | 74.8 | 77.7 | 82.4 |
| Comparative example 2 | Sample 2A | 11.7 | 30.1 | 46.7 | 65.9 | 72.4 | 76.5 | 81.9 |
| Comparative | Sample | 11.5 | 30 | 39.3 | 50.6 | 55.8 | 59.2 | 64.1 |
| example 3 | 7A | | | | | | | |
| Comparative example 4 | Sample 8A | 19.8 | 34.4 | 43 | 55.4 | 61.3 | 65.6 | 72.2 |

The dissolution results of Table 5 and FIG. 2 show that in phosphate buffer at pH6.8, when the D90 of spirocyclic aryl phosphorus oxide is in the range of 40.3 µm to 79.6 µm and the D50 is in the range of 13.0 µm to 23.8 µm, the dissolution results are substantially the same, and there is no significant difference. Compared with dissolution data of other particle sizes, spirocyclic aryl phosphorus oxides with D90 in the range of 40.3 µm to 79.6 µm and D50 in the range of 13.0 µm to 23.8 µm are dissolved out most rapidly from pharmaceutical compositions. When the particle size of D90 is less than 40.3 µm or D50 is less than 13.0 µm, the dissolution gradually becomes slower, and when D90 is more than 79.6 µm or D50 is more than 23.8 µm, the dissolution is also slower.

The dissolution results of Table 6 and FIG. 3 show that in the medium of purified water + 0.5% Tween 20, the dissolution rates of spirocyclic aryl phosphorus oxide are substantially the same when the D90 is in the range of 11.6 µm to 79.6 µm and the D50 is in the range of 5.4 µm to 23.8 µm, and the dissolution rate decreases when the particle size D90 of raw material is more than 100 µm or the D50 is more than 40 µm. Referring to Tables 5 and 6, spirocyclic aryl phosphorus oxides have good dissolution performance in the medium of phosphate buffer at pH6.8 and purified water + 0.5% Tween 20 when the D90 is in the range of 40.3 µm to 79.6 µm and the D50 is in the range of 13.0 µm to 23.8 µm.

### Investigation on the stability of pharmaceutical composition

### 1) Influencing factor test

The pharmaceutical composition prepared in Example 2 of the present invention was selected to investigate the generation of total impurities in the sample under the conditions of light, high temperature (60 °C or 40 °C) and high humidity (Rh90%±5% or Rh75%±5%). The calculation of total impurities (%) is obtained by determining the peak area ratio of the samples to be tested and the reference samples using LC.

**Table 7 Data of influencing factors of spirocyclic aryl phosphorus oxide tablets**

| **Influencing factors** | **Index** | **0 day** | **5 days** | **10 days** | **30 days** |
|---|---|---|---|---|---|
| Light | Total impurity (%) | none detected | 0.15 | 0.08 | 0.26 |
| High temperature (60 °C) | Total impurity (%) | none detected | 0.16 | 0.19 | 0.29 |
| High temperature (40 °C) | Total impurity (%) | none detected | 0.11 | 0.14 | 0.18 |
| High humidity test (RH90% ±5%) | Total impurity (%) | none detected | 0.10 | 0.15 | 0.11 |
| | Loss on drying (%) | 3.3 | 7.8 | 7.8 | 8.7 |
| High humidity test (RH75% ±5%) | Total impurity (%) | none detected | 0.15 | 0.10 | 0.11 |
| | Loss on drying (%) | 3.3 | 6.9 | 7.0 | 7.3 |

According to the results in the table, the related ingredients in the pharmaceutical composition in Example 2 increased slightly under the conditions of light and high temperature, but the content was low. Although the moisture increased a lot under high humidity, the related ingredients remained substantially unchanged. Therefore, the pharmaceutical composition provided by the present application has good stability under the conditions of high temperature and high humidity.

### 2) Accelerated stability test

Sample 1A, sample 3A and sample 4A of pharmaceutical compositions prepared in Comparative Example 1, Example 1 and Example 2 of the present invention were selected to investigate the formation of related ingredients (single impurity and total impurity) in the samples at 40°C and Rh75% respectively.

**Table 8 Accelerated stability data**

| **Time** | **Sample 1A (with desiccant)** | | | **Sample 1A (without desiccant)** | | | **Sample 3A (with desiccant)** | | | **Sample 4A (with desiccant)** | | |
|---|---|---|---|---|---|---|---|---|---|---|---|---|
| | Impurity 1(%) | Other single impur ity (%) | Total impur ity (%) | Impurity 1(%) | Other single impur ity (%) | Total impu rity (%) | Impurity 1(% ) | Other single impur ity (%) | Totalimpur ity (%) | Impurity 1(%) | Other single impur ity (%) | Total impu rity (%) |
| 0 day | 0.01 | 0.03 | 0.08 | 0.55 | None detect ed | 0.55 | 0.03 | 0.04 | 0.13 | 0.02 | 0.03 | 0.11 |
| Accelerate for 1 month exposure | / | / | / | / | / | / | 0.11 | 0.05 | 0.24 | 0.07 | 0.04 | 0.2 |
| Accelerate for 1 month seal | 0.01 | 0.03 | 0.13 | 0.56 | 0.05 | 0.61 | 0.07 | 0.04 | 0.23 | 0.06 | 0.04 | 0.16 |
| Accelerate for 2 months | 0.05 | 0.03 | 0.13 | 0.57 | None detect ed | 0.57 | 0.08 | 0.04 | 0.18 | 0.08 | 0.06 | 0.22 |
| Accelerate for 3 months | 0.09 | 0.04 | 0.17 | 0.58 | None detect ed | 0.58 | 0.1 | 0.05 | 0.2 | 0.09 | 0.05 | 0.21 |
| Accelerate for 6 months | 0.1 | 0.05 | 0.19 | 0.58 | 0.07 | 0.58 | 0.14 | 0.07 | 0.22 | 0.14 | 0.08 | 0.24 |

| | | | | | | | | | | | | |
|---|---|---|---|---|---|---|---|---|---|---|---|---|
| Note: the "/" in Table 8 indicates that there is no corresponding parameter. | | | | | | | | | | | | |

Sample 1A (with desiccant), sample 3A (with desiccant) and sample 4A (with desiccant) were all added with desiccant in the process of product stability placement. During the process, the stability was substantially the same, and the growth trend of related ingredients was substantially the same. Sample 1A (without desiccant) was not added with desiccant during the placement process, the related ingredients were more in 0 day, but did not change much in the later stage. Compared with the stability data of sample 1A with and without desiccant, the results showed that the total impurity content was significantly lower after adding desiccant.

### Clinical trials

### 1) Prescription

| **Raw materials** | **Sample 9A** | **Sample 10A** |
|---|---|---|
| Unit | mg/tablet | mg/tablet |
| spirocyclic aryl phosphorus oxide (D90 = 62 µm, D50 = 23.8 µm, D10 = 5.0 µm) | 30 | 60 |
| microcrystalline cellulose | 23.4 | 46.8 |
| pregelatinized starch | 55.2 | 110.4 |
| sodium carboxymethyl starch | 6.0 | 12.0 |
| hydroxypropyl methylcellulose | 3.6 | 7.2 |
| micronized silica gel | 0.6 | 1.2 |
| magnesium stearate | 1.2 | 2.4 |
| weight of plain tablet | 120 | 240 |
| Opadry coating powder (white) | 3.6 | 7.2 |

2) The preparation processes of pharmaceutical composition samples 9A and 10A is shown in Example 1.

3) Sample 9A and sample 10A were taken for clinical trials according to the following dosages. The data are shown in Table 9 below.

**Table 9 Clinical trial data of spirocyclic aryl phosphorus oxide tablets**

| **Administration dose** | **60mg** | **90mg** | **120mg** | **180mg** |
|---|---|---|---|---|
| Administration mode | 60mg/1 tablet | 30mg/1 tablet + 60mg/1 tablet | 60mg/2 tablets | 60mg/3 tablets |
| Cₘₐₓ (nM) | 274.32 | 356.47 | 489.22 | 369.00 |
| Tₘₐₙ (hr) | 1.328 | 1.685 | 1.808 | 2.288 |
| T_{1/2} (hr) | 25.56 | 21.70 | 21.87 | 23.53 |
| AUC₀₋ₜ (nM.hr) | 3377.43 | 4852.29 | 7134.80 | 5835.07 |
| AUC_{0-∞} (nM.hr) | 3778.77 | 5575.31 | 7437.80 | 6463.01 |
| MRT_{0-∞} (hr) | 28.00 | 23.79 | 25.20 | 27.44 |

According to the analysis of clinical data, AUC_{0-∞} substantially increased linearly when the dosage was increased from 60 mg to 120 mg, and increased by less than 1.5 times when the dosage was increased from 60 mg to 90 mg. Relatively speaking, the bioavailability of 60 mg was slightly higher in vivo. However, there is no significant difference in bioavailability between 60 mg and 120 mg dosages, and there is no statistically significant differences. However, the bioavailability of 180 mg dose group was lower than that of 120 mg dose group, indicating that the bioavailability in vivo did not increase with the increase of dosage.

## Claims

1. A pharmaceutical composition, comprising a spirocyclic aryl phosphorus oxide or a pharmaceutically acceptable salt thereof as an active ingredient, and a pharmaceutically acceptable carrier, wherein, the spirocyclic aryl phosphorus oxide has the following structure: 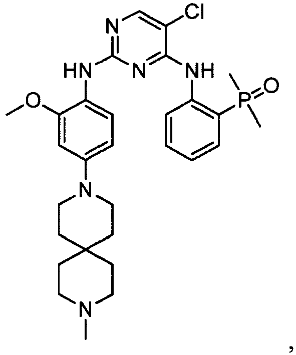 and
the D90 of the spirocyclic aryl phosphorus oxide or a pharmaceutically acceptable salt thereof is in a range of 40.3 µm to 79.6 µm, wherein the D90 is the particle size corresponding to the cumulative volume distribution percentage of the spirocyclic aryl phosphorus oxide or a pharmaceutically acceptable salt thereof reaching 90% measured from a small particle size,
the D50 of the spirocyclic aryl phosphorus oxide or a pharmaceutically acceptable salt thereof is in a range of 13.0 µm to 23.8 µm, wherein the D50 is the particle size corresponding to the cumulative volume distribution percentage of the spirocyclic aryl phosphorus oxide or a pharmaceutically acceptable salt thereof reaching 50% measured from a small particle size; and
the pharmaceutically acceptable carrier includes a filler, a disintegrant, an adhesive, a glidant and a lubricant.

2. The pharmaceutical composition according to claim 1, wherein, the D50 of the spirocyclic aryl phosphorus oxide or a pharmaceutically acceptable salt thereof is in a range of 13.0 µm to 18.2 µm.

3. The pharmaceutical composition according to claim 1 or 2, wherein, the spirocyclic aryl phosphorus oxide is in crystal form A.

4. The pharmaceutical composition according to claim 1, wherein, the filler is selected from the group consisting of starch, mannitol, sorbitol, microcrystalline cellulose, lactose, pregelatinized starch and inorganic salts or a combination thereof, preferably wherein, the filler includes a combination of microcrystalline cellulose and pregelatinized starch.

5. The pharmaceutical composition according to claim 1, wherein, the disintegrant is selected from the group consisting of dry starch, sodium carboxymethyl starch, hydroxypropyl starch, low substituted hydroxypropyl cellulose, crospovidone and croscarmellose sodium or a combination thereof, preferably wherein, the disintegrant includes sodium carboxymethyl starch.

6. The pharmaceutical composition according to claim 1, wherein, the adhesive is selected from the group consisting of distilled water, ethanol, starch paste, powdered sugar and syrup, hydroxypropyl methylcellulose, povidone, hydroxypropyl cellulose, methyl cellulose, ethyl cellulose and sodium carboxymethyl cellulose or a combination thereof, preferably wherein, the adhesive includes hydroxypropyl methylcellulose.

7. The pharmaceutical composition according to claim 1, wherein, the glidant is selected from the group consisting of micronized silica gel and talc or a combination thereof, preferably wherein, the glidant includes micronized silica gel.

8. The pharmaceutical composition according to claim 1, wherein, the lubricant is selected from the group consisting of magnesium stearate, hydrogenated vegetable oil, polyethylene glycol, dodecyl magnesium sulfate and sodium stearyl fumarate or a combination thereof, preferably wherein, the lubricant includes magnesium stearate.

9. The pharmaceutical composition according to any one of claims 1-8, wherein, the pharmaceutical composition is a tablet.

10. The pharmaceutical composition according to claim 9, wherein, the pharmaceutical composition is a film-coated tablet.

11. The pharmaceutical composition according to any one of claims 4-10, wherein, the components each in percentage by weight are: 1% to 50% of spirocyclic aryl phosphorus oxide or a pharmaceutically acceptable salt thereof, 1% to 90% of the filler, 1% to 10% of the disintegrant, 0.1% to 1% of the glidant and 0.5% to 5% of the lubricant, preferably wherein, the components each in percentage by weight are: 5.0% of spirocyclic aryl phosphorus oxide or a pharmaceutically acceptable salt thereof, 26.0% of microcrystalline cellulose, 59.5% of pregelatinized starch, 5% of sodium carboxymethyl starch, 3.0% of hydroxypropyl methylcellulose, 0.5% of micronized silica gel and 1% of magnesium stearate.

12. A method for preparing the pharmaceutical composition according to claim 1, comprising the following steps:
1) mixing spirocyclic aryl phosphorus oxide or a pharmaceutically acceptable salt thereof with internal excipients including a filler, an adhesive and a disintegrant, and then granulating;
2) adding other excipients including a filler, a disintegrant and a lubricant, and then mixing; and
3) tabletting and coating.

13. The pharmaceutical composition according to any one of claims 1-11 or the pharmaceutical composition prepared according to the method of claim 12 for use in treating cancer.

14. The pharmaceutical composition for use according to claim 13, wherein the cancer includes non-small cell lung cancer, lymphoma, non-Hodgkin lymphoma, ovarian cancer, cervical cancer, prostate cancer, colorectal cancer, breast cancer, pancreatic cancer, glioma, glioblastoma, leukemia, endometrial carcinoma, lung cancer, hepatocellular carcinoma, gastric carcinoma, gastrointestinal stromal tumor, acute myeloid leukemia, cholangiocarcinoma, renal carcinoma, thyroid cancer, anaplastic large cell lymphoma, mesothelioma, multiple myeloma, and melanoma, preferably wherein the cancer is non-small cell lung cancer.

## Patentansprüche

1. Eine pharmazeutische Zusammensetzung, umfassend ein spirozyklisches Arylphosphoroxid oder ein pharmazeutisch verträgliches Salz davon als Wirkstoff und einen pharmazeutisch akzeptablen Träger, wobei das spirozyklische Arylphosphoroxid die folgende Struktur aufweist: 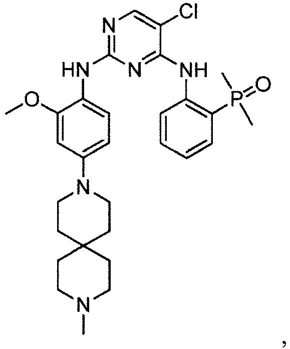 und
der D90-Wert des spirozyklischen Arylphosphoroxids oder eines pharmazeutisch akzeptablen Salzes davon im Bereich von 40,3 µm bis 79,6 µm liegt, wobei der D90-Wert die Partikelgröße ist, die dem kumulativen Volumenverteilungsanteil des spirozyklischen Arylphosphoroxids oder eines pharmazeutisch akzeptablen Salzes davon entspricht, der 90 % erreicht, gemessen ausgehend von einer kleinen Partikelgröße,
der D50-Wert des spirozyklischen Arylphosphoroxids oder eines pharmazeutisch akzeptablen Salzes davon im Bereich von 13,0 µm bis 23,8 µm liegt, wobei der D50-Wert die Partikelgröße ist, die dem kumulativen Volumenverteilungsanteil des spirozyklischen Arylphosphoroxids oder eines pharmazeutisch akzetablen Salzes davon entspricht, der 50 % erreicht, gemessen ausgehend von einer kleinen Partikelgröße; und
der pharmazeutisch akzeptable Träger ein Füllmittel, ein Sprengmittel, ein Bindemittel, ein Gleitmittel und ein Schmiermittel umfasst.

2. Pharmazeutische Zusammensetzung nach Anspruch 1, wobei der D50-Wert des spirozyklischen Arylphosphoroxids oder eines pharmazeutisch akzeptablen Salzes davon im Bereich von 13,0 µm bis 18,2 µm liegt.

3. Pharmazeutische Zusammensetzung nach Anspruch 1 oder 2, wobei das spirozyklische Arylphosphoroxid in der Kristallform A vorliegt.

4. Pharmazeutische Zusammensetzung nach Anspruch 1, wobei das Füllmittel aus der Gruppe ausgewählt ist, die aus Stärke, Mannit, Sorbit, mikrokristalliner Cellulose, Lactose, vorgelatinierter Stärke und anorganischen Salzen oder einer Kombination davon besteht, wobei das Füllmittel vorzugsweise eine Kombination aus mikrokristalliner Cellulose und vorgelatinierter Stärke enthält.

5. Pharmazeutische Zusammensetzung nach Anspruch 1, wobei das Sprengmittel aus der Gruppe ausgewählt ist, die aus trockener Stärke, Natriumcarboxymethylstärke, Hydroxypropylstärke, niedrigsubstituierter Hydroxypropylcellulose, Crospovidon und Croscarmellose-Natrium oder einer Kombination davon besteht, wobei das Sprengmittel vorzugsweise Natriumcarboxymethylstärke enthält.

6. Pharmazeutische Zusammensetzung nach Anspruch 1, wobei das Bindemittel aus der Gruppe ausgewählt ist, die aus destilliertem Wasser, Ethanol, Stärkepaste, Puderzucker und Sirup, Hydroxypropylmethylcellulose, Povidon, Hydroxypropylcellulose, Methylcellulose, Ethylcellulose und Natriumcarboxymethylcellulose oder einer Kombination davon besteht, wobei das Bindemittel vorzugsweise Hydroxypropylmethylcellulose enthält.

7. Pharmazeutische Zusammensetzung nach Anspruch 1, wobei das Gleitmittel aus der Gruppe ausgewählt ist, die aus mikronisiertem Silikagel und Talkum oder einer Kombination davon besteht, wobei das Gleitmittel vorzugsweise mikronisiertes Silikagel enthält.

8. Pharmazeutische Zusammensetzung nach Anspruch 1, wobei das Schmiermittel aus der Gruppe ausgewählt ist, die aus Magnesiumstearat, hydriertem Pflanzenöl, Polyethylenglykol, Dodecylmagnesiumsulfat und Natriumstearylfumarat oder einer Kombination davon besteht, wobei das Schmiermittel vorzugsweise Magnesiumstearat enthält.

9. Pharmazeutische Zusammensetzung nach einem der Ansprüche 1 bis 8, wobei die pharmazeutische Zusammensetzung eine Tablette ist.

10. Pharmazeutische Zusammensetzung nach Anspruch 9, wobei die pharmazeutische Zusammensetzung eine filmbeschichtete Tablette ist.

11. Pharmazeutische Zusammensetzung nach einem der Ansprüche 4 bis 10, wobei die Komponenten jeweils in Gewichtsprozent wie folgt vorliegen: 1% bis 50% spirozyklisches Arylphosphoroxid oder ein pharmazeutisch akzeptables Salz davon, 1% bis 90% des Füllmittels, 1% bis 10% des Sprengmittels, 0,1% bis 1% des Gleitmittels und 0,5% bis 5% des Schmiermittels, wobei vorzugsweise die Komponenten jeweils in Gewichtsprozent wie folgt vorliegen: 5,0% spirozyklisches Arylphosphoroxid oder ein pharmazeutisch akzeptables Salz davon, 26,0% mikrokristalline Cellulose, 59,5% vorgelatinierte Stärke, 5% Natriumcarboxymethylstärke, 3,0% Hydroxypropylmethylcellulose, 0,5% mikronisiertes Silicagel und 1% Magnesiumstearat.

12. Ein Verfahren zur Herstellung der pharmazeutischen Zusammensetzung nach Anspruch 1, umfassend die folgenden Schritte:
1) Mischen von spirozyklischem Arylphosphoroxid oder eines pharmazeutisch akzeptablen Salzes davon mit internen Hilfsstoffen, einschließlich eines Füllmittels, eines Bindemittels und eines Sprengmittels, und anschließendes Granulieren;
2) Hinzufügen weiterer Hilfsstoffe, einschließlich eines Füllmittels, eines Sprengmittels und eines Schmiermittels, und anschließendes Mischen; und
3) Tablettieren und Beschichten.

13. Pharmazeutische Zusammensetzung gemäß einem der Ansprüche 1 bis 11 oder die nach dem Verfahren gemäß Anspruch 12 hergestellte pharmazeutische Zusammensetzung zur Verwendung bei der Behandlung von Krebs.

14. Pharmazeutische Zusammensetzung zur Verwendung gemäß Anspruch 13, wobei der Krebs nicht-kleinzelligen Lungenkrebs, Lymphom, Non-Hodgkin-Lymphom, Eierstockkrebs, Gebärmutterhalskrebs, Prostatakrebs, Darmkrebs, Brustkrebs, Bauchspeicheldrüsenkrebs, Gliom, Glioblastom, Leukämie, Endometriumkarzinom, Lungenkrebs, Leberzellkarzinom, Magenkarzinom, gastrointestinalen Stromatumor, akute myeloische Leukämie, Cholangiokarzinom, Nierenkarzinom, Schilddrüsenkrebs, anaplastisches großzelliges Lymphom, Mesotheliom, multiples Myelom und Melanom umfasst, wobei der Krebs vorzugsweise nichtkleinzelliger Lungenkrebs ist.

## Revendications

1. Composition pharmaceutique, comprenant un oxyde de phosphore aryle spirocyclique ou un sel pharmaceutiquement acceptable de celui-ci en tant que principe actif, et un support pharmaceutiquement acceptable, dans laquelle l'oxyde de phosphore aryle spirocyclique présente la structure suivante : 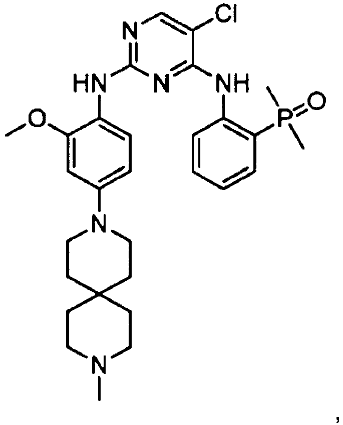 et
le D90 de l'oxyde de phosphore aryle spirocyclique ou d'un sel pharmaceutiquement acceptable de celui-ci se situe dans une plage de 40,3 µm à 79,6 µm, dans lequel le D90 est la taille de particule correspondant au pourcentage de distribution volumique cumulée de l'oxyde de phosphore aryle spirocyclique ou d'un sel pharmaceutiquement acceptable de celui-ci atteignant 90 % mesurée à partir d'une taille des petites particules,
le D50 de l'oxyde de phosphore aryle spirocyclique ou d'un sel pharmaceutiquement acceptable de celui-ci se situe dans une plage de 13,0 µm à 23,8 µm, dans lequel le D50 est la taille de particule correspondant au pourcentage de distribution volumique cumulée de l'oxyde de phosphore aryle spirocyclique ou d'un sel pharmaceutiquement acceptable de celui-ci atteignant 50 % mesurée à partir d'une taille des petites particules ; et
le support pharmaceutiquement acceptable comprend un agent de charge, un désintégrant, un liant, un agent d'écoulement et un lubrifiant.

2. Composition pharmaceutique selon la revendication 1, dans laquelle le D50 de l'oxyde de phosphore aryle spirocyclique ou d'un sel pharmaceutiquement acceptable de celui-ci se situe dans une plage de 13,0 µm à 18,2 µm.

3. Composition pharmaceutique selon la revendication 1 ou 2, dans laquelle l'oxyde de phosphore aryle spirocyclique est sous forme cristalline A.

4. Composition pharmaceutique selon la revendication 1, dans laquelle l'agent de charge est choisi dans le groupe constitué par l'amidon, le mannitol, le sorbitol, la cellulose microcristalline, le lactose, l'amidon prégélatinisé et les sels inorganiques ou une combinaison de ceux-ci, de préférence dans laquelle l'agent de charge comprend une combinaison de cellulose microcristalline et d'amidon prégélatinisé.

5. Composition pharmaceutique selon la revendication 1, dans laquelle le désintégrant est choisi dans le groupe constitué par l'amidon sec, le carboxyméthylamidon sodique, l'amidon hydroxypropylé, l'hydroxypropylcellulose faiblement substituée, la crospovidone et la croscarmellose sodique ou une combinaison de ceux-ci, de préférence dans laquelle le désintégrant comprend du carboxyméthylamidon sodique.

6. Composition pharmaceutique selon la revendication 1, dans laquelle le liant est choisi dans le groupe constitué par l'eau distillée, l'éthanol, la pâte d'amidon, le sucre en poudre et le sirop, l'hydroxypropylméthylcellulose, la povidone, l'hydroxypropylcellulose, la méthylcellulose, l'éthylcellulose et la carboxyméthylcellulose de sodium ou une combinaison de ceux-ci, de préférence dans laquelle le liant comprend de l'hydroxypropylméthylcellulose.

7. Composition pharmaceutique selon la revendication 1, dans laquelle l'agent d'écoulement est choisi dans le groupe constitué par le gel de silice micronisé et le talc ou une combinaison de ceux-ci, de préférence dans laquelle l'agent d'écoulement comprend du gel de silice micronisé.

8. Composition pharmaceutique selon la revendication 1, dans laquelle le lubrifiant est choisi dans le groupe constitué par le stéarate de magnésium, une huile végétale hydrogénée, le polyéthylène glycol, le dodécyl sulfate de magnésium et le stéaryl fumarate de sodium ou une combinaison de ceux-ci, de préférence dans laquelle le lubrifiant comprend du stéarate de magnésium.

9. Composition pharmaceutique selon l'une quelconque des revendications 1 à 8, dans laquelle la composition pharmaceutique est un comprimé.

10. Composition pharmaceutique selon la revendication 9, dans laquelle la composition pharmaceutique est un comprimé pelliculé.

11. Composition pharmaceutique selon l'une quelconque des revendications 4 à 10, dans laquelle les constituants, chacun en pourcentage en poids, sont : 1 % à 50 % d'oxyde de phosphore aryle spirocyclique ou d'un sel pharmaceutiquement acceptable de celui-ci, 1 % à 90 % de l'agent de charge, 1 % à 10 % du désintégrant, 0,1 % à 1 % de l'agent d'écoulement et 0,5 % à 5 % du lubrifiant, de préférence dans laquelle, les constituants sont chacun en pourcentage en poids : 5,0 % d'oxyde de phosphore aryle spirocyclique ou d'un sel pharmaceutiquement acceptable de celui-ci, 26,0 % de cellulose microcristalline, 59,5 % d'amidon prégélatinisé, 5 % de carboxyméthylamidon sodique, 3,0 % d'hydroxypropylméthylcellulose, 0,5 % de gel de silice micronisé et 1 % de stéarate de magnésium.

12. Procédé de préparation de la composition pharmaceutique selon la revendication 1, comprenant les étapes suivantes :
1) le mélange de l'oxyde de phosphore aryle spirocyclique ou d'un sel pharmaceutiquement acceptable de celui-ci avec des excipients internes comprenant un agent de charge, un liant et un désintégrant, puis la granulation ;
2) l'ajout d'autres excipients comprenant un agent de charge, un désintégrant et un lubrifiant, puis le mélange ; et
3) la compression en comprimés et le pelliculage.

13. Composition pharmaceutique selon l'une quelconque des revendications 1 à 11 ou composition pharmaceutique préparée selon le procédé de la revendication 12 pour une utilisation dans le traitement du cancer.

14. Composition pharmaceutique pour une utilisation selon la revendication 13, dans laquelle le cancer comprend le cancer du poumon non à petites cellules, le lymphome, le lymphome non hodgkinien, le cancer de l'ovaire, le cancer du col de l'utérus, le cancer de la prostate, le cancer colorectal, le cancer du sein, le cancer du pancréas, le gliome, le glioblastome, la leucémie, le carcinome de l'endomètre, le cancer du poumon, le carcinome hépatocellulaire, le carcinome gastrique, la tumeur stromale gastro-intestinale, la leucémie myéloïde aiguë, le cholangiocarcinome, le carcinome rénal, le cancer de la thyroïde, le lymphome anaplasique à grandes cellules, le mésothéliome, le myélome multiple et le mélanome, de préférence dans laquelle le cancer est le cancer du poumon non à petites cellules.
